Europäisches Patentamt

European Patent Office

Office européen des brevets

(11) Publication number: **0 233 019 B1**

# EUROPEAN PATENT SPECIFICATION

(45) Date of publication of patent specification: **23.12.92**

(51) Int. Cl.[5]: **C12N 15/52**, C12N 1/20, C12P 19/06, //(C12N1/20, C12R1:64,1:19)

(21) Application number: **87300797.5**

(22) Date of filing: **29.01.87**

(54) Recombinant DNA plasmid for xanthan gum synthesis.

(30) Priority: **06.02.86 US 826537**
**18.09.86 US 908544**

(43) Date of publication of application:
**19.08.87 Bulletin 87/34**

(45) Publication of the grant of the patent:
**23.12.92 Bulletin 92/52**

(84) Designated Contracting States:
**AT CH DE FR GB IT LI NL**

(56) References cited:
**WO-A-87/05938**

**INTERNATIONAL JOURNAL OF BIOLOGICAL MACROMOLECULES, vol. 8, December 1986, pages 372-374, Butterworth & Co. (Publishers) Ltd, London, GB; G.C. BARRERE et al.: "Molecular cloning of genes involved in the production of the extracellular poly-saccharide xanthan by Xanthomonas campestris pv. campestris"**

(73) Proprietor: **MERCK & CO. INC.**
**126, East Lincoln Avenue P.O. Box 2000 Rahway New Jersey 07065-0900(US)**

(72) Inventor: **Cleary, Joseph M.**
**8042 Lake Adion Drive**
**San Diego California 92119(US)**
Inventor: **Rosen, Ira-G.**
**4088 Mount Acadia Boulevard**
**San Diego California 92111(US)**
Inventor: **Harding, Nancy E.**
**9028 Ellingham St.**
**San Diego California 92119(US)**
Inventor: **Cabanas, Debra K.**
**2980 First Avenue Apt. 2C**
**San Diego California 92103(US)**

(74) Representative: **Hesketh, Alan, Dr.**
**European Patent Department Merck & Co., Inc. Terlings Park Eastwick Road Harlow Essex, CM20 2OR(GB)**

ABSTRACTS OF THE ANNUAL MEETING OF THE AMERICAN SOCIETY OF MICROBIOLO-GY, Washington D.C., 23rd-28th March 1986, page 272, abstract no. O-64; N.E. HARDING et al.: "Genetic and physical analysis of cloned xanthan gum biosynthetic genes from Xanthomonas campestris"

CHEMICAL ABSTRACTS, vol. 99, no. 7, 15th August 1983, page 247, abstract no. 50041d, Columbus, Ohio, US; Y.H. TSENG et al.: "Pleiotropic effects in nonmucoid mutants of Xanthomonas campestris pv. oryzae" & PROC. NATL. SCI. COUNC., REPUB. CHINA, PART B 1983, 7(1), 44-50

THE EMBO JOURNAL, vol. 3, no. 13, 1984, pages 3323-3328, IRL Press Ltd, Oxford, GB; M.J. DANIELS et al.: "Cloning of genes involved in pathogenicity of Xanthomonas campestris pv. campestris using the broad host range cosmid pLAFR1"

**Description**

BACKGROUND OF THE INVENTION

1. Field of the Invention

The present invention relates to a method for increasing the yield of xanthan gum during fermentation of the microorganism Xanthomonas campestris by utilizing in the microorganism, a multi-copy recombinant DNA plasmid containing some genetic information essential for xanthan gum production.

2. Brief Description of Disclosures in the Art

Xanthan gum is the common name for the water-soluble microbial exopolysaccharide, comprised of D-glucose, D-mannose, D-glucuronic acid, acetic acid and pyruvic acid, produced by the aqueous aerobic fermentation of Xanthomonas campestris in the presence of D-glucose or other carbon sources. Aqueous solutions of the polysaccharide exhibit a) high viscosity at low concentration, (b) high pseudoplasticity levels (i.e. reversible decrease in viscosity with increase in shear rate) and (c) atypical polysaccharide insensitivity to a wide range of salt concentration, pH and temperatures.

Because of these outstanding solution properties, xanthan gum has become established as a leading thickening, suspending, and stabilizing agent in the food industry and in industrial technology.

Because of the industrial importance of this polysaccharide, for example in the oil industry, where xanthan gum finds use as a mobility control agent in enhanced oil recovery, new methods and microorganisms are constantly being searched for, in an effort to increase production and improve the rate and yield of xanthan gum during fermentation and to enhance its physical properties, particularly its thermostability under acid conditions. One focus of this effort is directed to the use of recombinant DNA technology in fermentation research.

The Cohen-Boyer patents, USP 4,237,224 and 4,468,464 were the first major broad disclosures in the field of recombinant DNA technology, particularly relating to the introduction of a foreign gene into a cloning vector for transformation into a suitable bacterial host, e.g. E. coli.

Other disclosures in the recombinant DNA field include: USP 4,332,900, disclosing cointegrate plasmids of Streptomyces and Escherichia; USP 4,370,417, (disclosing recombinant DNA coding for tissue plasminogen activator; U.S.P. 4,418,194) disclosing broad bacterial host plasmid cloning vehicles; and U.S.P. 4,520,103 disclosing microbial synthesis of indigo in indole-free media, in which transformed cells facilitate enzyme catalyzed oxidative transformation of cellular-produced indole.

Further, U.S.P. 4,393,135, discloses an L-glutamic acid producing microorganism, containing a hybrid plasmid having inserted therein, a DNA fragment with genetic information controlling L-glutamic acid production, said fragment being derived from a donor strain of Escherichia coli, which is capable of producing high levels of L-glutamic acid.

Similarly, U.S.P. 4,391,907 (to the same assignee as above) discloses an L-valine producing microorganism containing a hybrid plasmid having inserted therein a DNA fragment with genetic information related to L-valine production which is derived from a donor strain of the genus Escherichia and is useful for the production of high levels of L-valine by fermentation.

Proc. Natl. Acad. Sci. USA, Vol. 77, No. 12, pp 7347-7351 (December 1980) describes the non-self-transmissible bacterial plasmid pRK290, which is a broad host range cloning vehicle that can be mobilized at high frequency into Gram-negative bacteria. Also disclosed is the kanamycin-resistant helper plasmid pRK2013 which functions to transcomplement the pRK290 for mobilization.

Further, Plasmid 13, pp 149-153 (1985), describes the plasmid pRK293, which serves as the cloning vector of choice for the subject chromosomal DNA fragment from Xanthomonas campestris described herein.

Furthermore, a Xanthomonas campestris mutant is described in U.S.P. 4,296,303 which produces a pyruvate-free xanthan gum useful in oil drilling in partially depleted reservoirs.

However, none of the references described above, specifically teach how to increase the yield of xanthan gum by use of recombinant DNA techniques employing a region of the genome of Xanthomonas campestris essential for gum production, or exopolysaccharide synthesis.

OBJECTS OF THE INVENTION

It is an object of the present invention to provide a fragment of DNA from gum-producing Xanthomonas campestris which contains some of the genetic information essential for preducing xanthan gum. It is a further object to incorporate the fragment into a suitable vector thereby providing a recombinant DNA plasmid. It is a further object to incorporate the recombinant DNA plasmid into a specific gum-forming Xanthomonas campestris strain to increase the yield of xanthan gum, which also possesses a higher pyruvate content. These and other objects of the present invention will be apparent from the following description.

SUMMARY OF THE INVENTION

It has been found that xanthan gum production by Xanthomonas campestris (NRRL B-1459, ATCC No. 13951) can be significantly increased during fermentation by introduction into the microorganism of a recombinant DNA plasmid containing some genetic information essential for xanthan gum production. Other plasmids having similar effects on the increase of xanthan gum production in X. campestris are also contemplated.

The constructed plasmid is a multi-copy plasmid constructed from cloning vector pRK293 and a 12.4 kb chromosomal DNA fragment from a region of the Xanthomonas campestris genome encoding genes essential for gum synthesis. The plasmid is initially transformed into E.coli and then transferred to Xanthomonas campestris via conjugal mating with the aid of pRK2013 (ATCC No. 37159) helper plasmid.

The recombinant DNA plasmids of the present invention increase the yield of xanthan gum during fermentation, restore gum-producing ability to several mutants lacking the essential genetic gum-producing information, may increase the pyruvate content of the resulting xanthan gum, and are stable during fermentation.

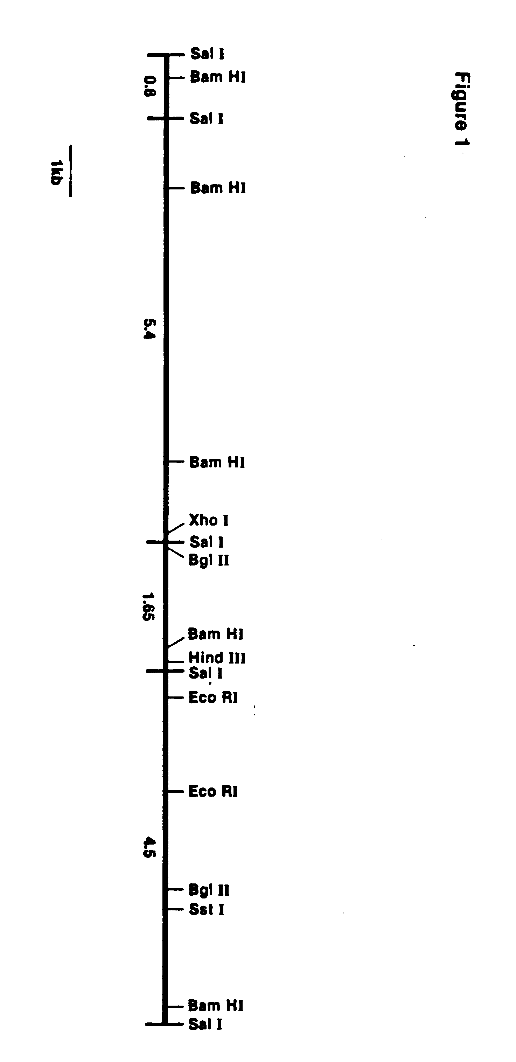

In accordance with this invention there is provided a double-stranded DNA fragment from the genome of a microorganism of the species Xanthomonas campestris, said fragment comprised of ca. 12.4 kb, having a restriction endonuclease map as depicted in Figure 1, and containing DNA sequences encoding genetic information essential for producing xanthan gum.

Further Provided are:

(1) a recombinant DNA plasmid covalently containing therein the double-stranded DNA fragment as defined above;

(2) a recombinant DNA plasmid covalently containing therein a) the double-stranded DNA fragment as defined above, and b) a plasmid DNA vector derived from the SalI cleavage of a Gram-negative bacterial DNA plasmid, said plasmid DNA vector being capable of replication in Xanthomonas campestris;

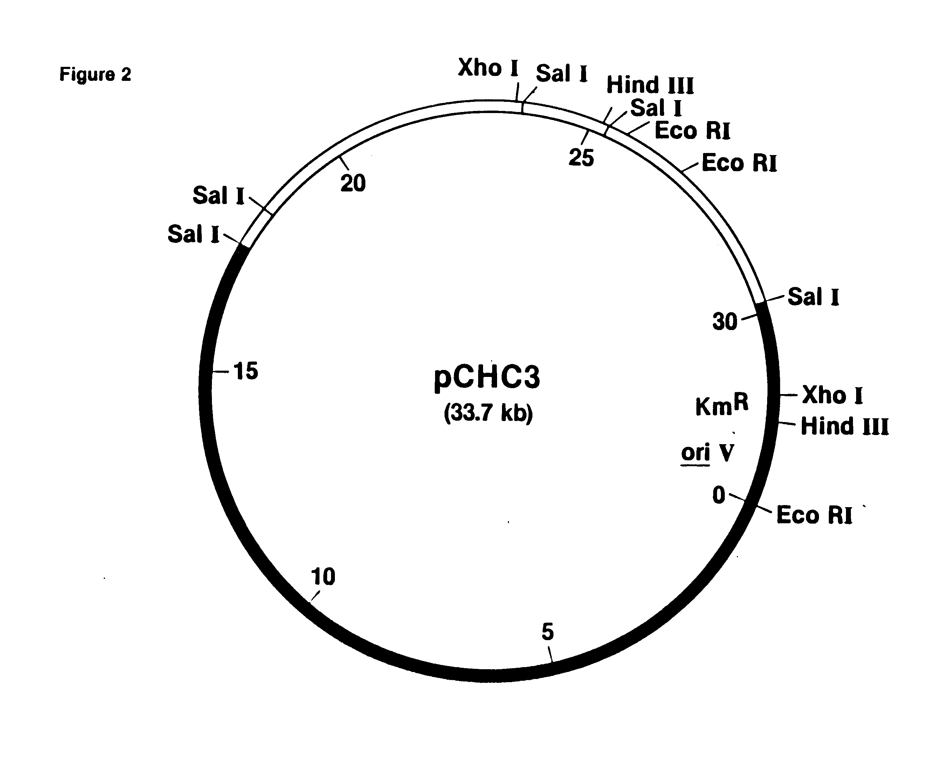

(3) a recombinant DNA Plasmid having a restriction endonuclease map as depicted in Figure 2, which upon introduction into a xanthan gum-producing microorganism of the species Xanthomonas campestris results in increased production of said xanthan gum; and

(4) a recombinant DNA plasmid having a restriction endonuclease map as depicted in Figure 2, which upon introduction into a xanthan gum-producing microorganism of the species Xanthomonas campestris results in the production of xanthan gum having a higher pyruvate content than that produced in the absence of said plasmid.

Also provided is a microorganism of the species Xanthomonas campestris, having the characteristics of ATCC No. 13951 (NRRL B-1459), having introduced therein at least one recombinant DNA plasmid as defined above, and being capable of increased xanthan gum production by fermentation in the presence of an aqueous buffered nutrient medium containing assimilable sources of carbon, nitrogen and inorganic substances, as contrasted to the same microorganism in the absence of said recombinant DNA plasmid.

BRIEF DESCRIPTION OF THE DRAWINGS

Figure 1 is a restriction endonuclease map of the 12.4 kb DNA fragment from Xanthomonas campestris chromosomal DNA.

Figure 2 is a restriction endonuclease map of circularized recombinant plasmid pCHC3. The solid bar indicates the original DNA of cloning vector pRK293 and the white bar indicates the inserted DNA fragment of Figure 1 being covalently linked at mutual SalI cleavage sites.

DETAILED DESCRIPTION OF THE INVENTION AND PREFERRED EMBODIMENTS

The chromosomal DNA fragment covalently contained within plasmid pCHC3 of the subject invention is present in E. coli., ATCC No. 53364. The plasmid can be inserted into Xanthomonas campestris NRRL B-1459, by transconjugation or transformation techniques as described herein. The claimed chromosomal DNA fragment, as well as the cloning vector pRK293, both of which together constitute plasmid pCHC3, can be obtained from the plasmid pCHC3 by cleavage with SalI restriction endonuclease by the procedures as described herein.

The subject chromosomal DNA fragment can also be obtained by isolating the total chromosomal DNA of Xanthomonas campestris according to analogous procedures in the art, as for example, T.W, Okita et al., J. Biol. Chem. 256, 6944-6952 (1981), which reference is hereby incorporated by reference for this particular purpose.

The total chromosomal DNA is then subjected to partial SalI digestion followed by velocity sedimentation on sucrose gradients according to the general procedure of Maniatis et al., Molecular Cloning, A Laboratory Manual, pp. 282-285, Cold Spring Harbor Laboratory (1982). Agarose gel electrophoresis using DNA size markers can be used to identify the 10-20 Kb SalI DNA fragment portion from which the 12.4 kb subject fragment is obtained by ligation into a suitable cloning vector and subsequent isolation and characterization. It will be understood that other restriction endonucleases may be useful in isolating the DNA sequences of the 12.4 kb fragment.

The restriction endonuclease map of the obtained subject DNA fragment in Figure 1 illustrates the length of the fragment as being 12.4 kb in length and the restriction enzyme sites which have been determined for SalI, BamHI, XhoI, BglII, HindIII, EcoRI , and SstI restriction enzymes. The fragment exists in double stranded configuration obtained from the partial SalI digestion.

The isolated DNA fragment contains genetic information essential to xanthan gum synthesis. This characteristic of the plasmid can be been shown by "complementation" experiments, "rescue" procedures, where the fragment, present in a suitable cloning vector, having a multiple copy number in the order of 2-20, is transferred by a transconjugation procedure to a mutant strain of Xanthomonas campestris, which is non-mucoid, i.e., non gum-producing, and results in restoration of the gum-forming ability in the transconjugant. These procedures may also be used to isolate mutants, gum-producing subfragments, or other subsets of the isolated fragment having the characteristic of restoring, after appropriate manipulations, the gum-forming ability in the transconjugate. In addition, DNA fragments larger than the 12.4 kb subject fragment may also have substantially similar effects on gum-producing ability, any of which may also be isolated, characterized, and analyzed by the methods of the present invention.

Further, the obtained subject DNA fragment possesses the ability to amplify the gum-producing ability of NRRL B-1459; i.e. increase the yield of xanthan gum in the range of 10 to 15%, as determined gravimetrically with the dried, alcohol-precipitated fermentation product, as contrasted with the microorganism in the absence of the plasmid-bearing fragment. This effect, also termed "gene-dosage effect", represents an important procedure for the commercial production of xanthan gum by fermentation.

Another characteristic of the fragment is its ability to increase the pyruvate content of the xanthan gum obtained from NRRL B-1459. The higher the pyruvate content, the greater the thermal stability of the gum, (Nisbet, et al., Second European Symposium on Enhanced Oil Recovery (1982)) which is a desirable feature, for example, when the gum is used in enhanced oil recovery operations under high temperature conditions, eg. 90-100°C. The increase in pyruvate content is generally in the order of about 30 to 50% of the amount of pyruvate present in xanthan gum produced from a strain lacking the plasmid bearing fragment.

The cloning vector, or plasmid vector desired in the recombinant DNA plasmid of the instant invention should desirably possess several basic characteristics:

1. The plasmid cloning vector should be relatively small, of the order 25 kb or less. Smaller plasmids during transformation can carry larger pieces of host DNA before the newly constructed plasmids become too large to transform efficiently.

2. The plasmid vector should have one unique restriction endonuclease site which is not located within an essential replication gene, preferably being a SalI restriction site. This site may be formed by use of another restriction enzyme, e.g. BamHI, followed by SalI linker generation and attachment.

3. The plasmid should have a host range replication system in Gram-negative bacteria, at least Xanthomonas campestris and E. coli. This is desirable because it may be necessary to perform some procedures in one organism and other procedures in others. For example, it is very difficult to transform Xanthomonas campestris with plasmid DNA but relatively easy to transform certain strains of E. coli.

4. The plasmid should have a marker allowing for selection of transformants, such as genes coding for antibiotic resistance, e.g. tetracycline, kanamycin, or ampicillin.

5. The recombinant plasmid should be capable of being introduced into the target cell by transformation, or mixing the strains containing the recombinant plasmid, helper plasmid and target organism (triparental mating) and selecting for target cells containing the plasmid, or by mating the helper plasmid into an E. coli carrying the plasmid and using this new strain carrying both plasmids to mate the plasmid into the target strain.

Examples of suitable cloning vectors exhibiting the above characteristics are those derived from IncP plasmids such as RK2, eg. pRK290, having ATCC No. 37168, (described by G. Ditta et al. in Proc. Natl. Acad. Sci. USA, Vol. 77, No. 12, pp. 7347-7351 (December 1980); pRK291, pRK292, pRK293, pRK310, pRK311, and the like, (described from G. Ditta et al. in Plasmid 13, pp. 149-153 (1985), or derived from IncQ plasmids such as RSF1010, e.g., pKT210, pKT248 (described by M. Bagdasarian, et al., in Gene, Vol. 16, pp. 237-247 (1981), in which all the plasmids described above being publicly available. Also operable and included within the instant invention is plasmid pVK101, having ATCC No. 37157.

Plasmid RK2 is a naturally occurring broad host range plasmid originally isolated from a clinical isolate. It is identical to plasmids R68, RP1 and RP4, isolated from Pseudomonas aeruginosa, as revealed by electron microscopic examination of heteroduplexes and restriction mapping.

Plasmid pRK2501 is prepared by making deletions of RK2 by using restriction enzymes. Plasmid pRK290 is isolated as a construct derived of segments of plasmid pRK2501 DNA and segments of RK2.

Plasmid pRK293 is derived from a natural in vivo mating event between plasmid pRK290 and pRK2501 to give a kanamycin resistant variant of pRK290 , namely pRK293. The preferred plasmid for use as cloning vector in the instant invention is pRK293, which is publicly available from University of California at San Diego.

The plasmid pRK293 possesses all of the characteristics listed above; it is relatively stable in Xanthomonas campestris during fermentation; it does not appear to cause any adverse effects on the ability of the DNA fragment to express the genes encoded for gene-dosage, complementation or increasing pyruvate content. The plasmid is a Gram-negative bacterial plasmid, has a copy number in Xanthomonas campestris of about 5-10, is non-self-transmissible, contains a single SalI restriction enzyme site in its tetracycline-resistant gene and is capable of replicating in Xanthomonas campestris.

Preparative amounts of plasmid vector DNA can be isolated from its E. coli host by any of several isolation procedures, for example, that of Kahn et al., "Methods in Enzymology" Vol. 68, pg. 268 (1979) or that of Currier and Nestor, Anal. Biochem., Vol. 76, pg. 431 (1976).

The insertion of the chromosomal DNA fragment into the plasmid cloning vector is accomplished by concurrently and separately conducting a partial digestion of the X. campestris chromosomal DNA, and a complete digestion of pRK293 with the restriction endonuclease, SalI. Other restriction endonucleases may also be used for this purpose by, e.g., adding linkers to the partially digested DNA to make the ends adaptable for use in pRK293.

The partial SalI digestion mixture of chromosomal DNA is subjected to velocity sedimentation and agarose gel electrophoresis to isolate the 10-20 kb chromosomal fragment portion.

This portion is then mixed with the singly SalI cleaved pRK293 and ligated, using appropriate conditions for ligation of plasmid and chromosomal DNA, as described, for example, by The Bethesda Research Laboratories Newsletter, Focus, Vol. 2, No. 3, (1979).

Following annealing and ligation, the recombinant plasmids, covalently containing the X. campestris chromosomal fragments, are introduced into E. coli under transforming conditions by the method of M. Dagert and S. Ehrlich in Gene, Vol. 6, pp. 23-28 (1979), hereby incorporated by reference for this particular purpose.

E. coli transformants are isolated by growth on kanamycin, which selects for growth of cells having acquired the plasmid vector's kanamycin resistance (Km$^R$) gene. Transformed E. coli cells containing recombinant plasmids are identified by the characteristic that insertion of chromosomal DNA into the SalI site contained within the tet gene renders those cells tetracycline sensitive (Tc$^S$). The total collection of Km$^R$ Tc$^S$ colonies thus obtained constitute the gene library which is used as the source for recombinant plasmids containing X. campestris chromosomal DNA fragments.

Transconjugation of plasmids from E. coli to Xanthomonas campestris can be accomplished by substantially the same triparental conjugation procedure described by Ditta et al. in Proc. Natl. Acad. Sci USA, Vol. 77, pp 7347 to 7351 (1980) involving helper plasmid pRK2013, having ATCC No. 37159.

The E. coli donor strains can be counterselected by using an X. campestris recipient strain which is resistant to an antibiotic such as rifampicin or streptomycin. Alternatively, auxotrophic donors can be counterselected by growth of the prototrophic X. campestris recipient on minimal medium.

Alternately, Xanthomonas campestris can be transformed directly by plasmids utilizing the general procedure of Dagert and Ehrlich, supra, which doesn't require the assistance of helper plasmid, but with greatly reduced efficiency.

Plasmids containing essential gum genes are identified bY their ability to restore gum synthesis to non-mucoid mutants of X. campestris.

The E. coli colonies, containing those plasmids giving rise to mucoid colonies from mutant non-mucoid strains of Xanthomonas campestris, are trans-conjugated with gum-producing NRRL B-1459, which is then subjected to shake-flask fermentation to determine if the trans-conjugated plasmid causes or elicits a gene dosage effect.

In this manner, the plasmid pCHC3 and other plasmids are isolated, each of which exhibits the ability to broadly complement several non-mucoid mutants, cause a significant gene-dosage effect on yield, and may demonstrate an ability to increase gum pyruvate content. The plasmid pCHC3 is illustrated in Figure 2, in which the restriction endonuclease map is depicted. Plasmids having related but different DNA sequences are also isolated by this procedure. The present invention encompasses these other plasmids, since they also have the characteristic of broadly complementing a variety of non-mucoid mutants of X. campestris.

E. coli JZ279, containing the recombinant DNA plasmid pCHC3 described above, is on deposit with the American Type and Tissue Culture Collection in Bethesda, Maryland, having the ATCC No. 53364, said deposit complying with the terms of the Budapest Treaty.

Also an aspect of the present invention is the microorganism Xanthomonas campestris, having the characteristics of NRRL B-1459, containing at least one recombinant DNA pCHC3 plasmid, and preferably being present by virtue of a multiple copy number, as described herein, which is capable of increased xanthan gum production during fermentation.

Processes for xanthan gum production by aerobic fermentation are well known in the art. Examples are the following United States Patents: 3,671,398; 3,433,708; 3,271,267; 3,594,280; 3,427,726; 3,251,749; 3,591,578; 3,391,061; 3,020,206; 3,481,889 and 3,391,060.

In general, the fermentations are carried out in aqueous buffered media containing assimilable sources of carbon, nitrogen and inorganic substances. The fermentation is usually carried out between 28 and 33°C and preferably around 30°C, under strong stirring and aeration conditions and buffered by means of buffering salts at a pH of about 7. A lower pH, due to the build-up of acid materials during fermentation, generally causes a decrease in the desirable properties of the obtained gum. Alternatively, buffering salts can be replaced by controlled addition of acid and base.

Examples of suitable carbon sources are D-glucose, corn syrup, hydrolyzed starch or other carbohydrates which contain a high proportion of D-glucose. Examples of suitable nitrogen sources are ammonia, ammonium salts, yeast or casein hydrolysates, soybean meal, distillers solubles and the like.

Xanthan gum produced in the process is recovered by conventional procedures and the fermentation is conducted in conventional apparatus.

The microorganism NRRL B-1459, containing the recombinant DNA plasmid described above, which is present in 2-20 copies, preferably being pCHC3, can be in the lyophilized form and/or in biologically pure form as obtained from the following Examples.

The following examples are illustrative of means of carrying out the instant invention and should not be construed as being limitations on the scope or spirit of the invention as claimed.

Bacterial Strains, Plasmids and Media Used in The Examples - X. campestris (mutant strain of NRRL B-1459) was used as a source of chromosomal DNA. Recombination-deficient E. coli JZ279, recA, lacY, galK, galT, metBl, trpR55, supE, supF, hsdR514 was used as the recipient for the gene library. The cloning vehicle, pRK293, (publicly available from University of California at San Diego) is a 21 kb Km$^R$, Tc$^R$ derivative of the broad host range plasmid RK2. (pRK2013 is also publicly available from the University of California at San Diego). Both E. coli and X. campestris were grown in a rich medium, TYE (tryptone-yeast extract medium of Miller, Experiments in Molecular Genetics pp. 352-355, Cold Spring Harbor Laboratory, Cold Spring Harbor, New York, 1972). Where appropriate, kanamycin at 50 μg/ml tetracycline at 6 μg/ml or rifampicin at 100 μg/ml was added to the media. Where noted, X. campestris was also grown on M9 minimal agar (Miller, cited above) or YM agar (Difco) to detect the mucoid phenotype.

EXAMPLE 1

Plasmids containing genes essential for xantham gum synthesis were isolated by introducing the gene library into non-mucoid mutants of X. campestris by conjugation and selecting those recombinant plasmids which complemented (restored gum synthesis).

Isolation of Plasmids Containing Essential Gum Synthesis Genes - The pooled clone bank was mated with each of three rifampicin resistant non-mucoid mutants of X. campestris. The vector pRK293 is not self-transmissible, but can be mobilized using another plasmid (in this case pRK2013, Ditta et al. cited above, 1980), which provides transfer functions in trans. After overnight incubation at 30°C on TYE agar (YT medium of Miller, cited above 1972), the conjugation mixtures were plated for single colonies on YM agar containing kanamycin and rifampicin. Among the Km$^R$ transconjugants a few mucoid colonies were obtained.

7

Several mucoid transconjugants from each mating were selected at random for further study. DNA was prepared from each by the cleared lysate technique (Kahn et al., cited above), then used to transform E. coli JZ279. These E. coli strains were used as donors in conjugal matings with several other X. campestris non-mucoid mutants.

Among the collection of recombinant plasmids thus obtained the plasmid designated pCHC3 was able to complement all but one of the non-mucoid mutants tested (9 of 10).

Physical analysis of the collection of complementing plasmids was performed by restriction endonuclease analysis and electrophoresis as described by Davis, et al., in Advanced Bacterial Genetics, A Manual for Genetic Engineering, Cold Spring Harbor Laboratory, Cold Spring Harbor, N.Y. (1980).

The physical map of pCHC3 was derived by single and double restriction enzyme digests (SalI, EcoRI, HindIII, SmaI, SstI and XhoI), and by comparison to restriction patterns of plasmids with inserts comprised of DNA overlapping that of pCHC3 and complementing one or more of the non-mucoid mutants. Plasmid pCHC3 is comprised of four SalI fragments of 5.4, 4.55, 1.65 and 0.8 kb inserted into the unique SalI site in the tet gene of the broad host range vector pRK293.

The physical mapping of these related plasmids indicated all the SalI fragments in the insert in pCHC3 were derived from the same contiguous region of X. campestris.

EXAMPLE 2

**DEMONSTRATION OF THE INCREASED XANTHAN GUM PRODUCTION BY X. CAMPESTRIS NRRL B-1459 CONTAINING THE PLASMID pCHC3.**

To demonstrate the utility of this plasmid with respect to the usefulness of this invention, a study of the fermentation yields of the X. campestris strain, NRRL B-1459, with and without the plasmid, pCHC3, was done. The results of a typical experiment are shown in Table I.

For this study, the plasmid pCHC3 was transferred by conjugation from E. coli JZ279 ATCC No. 53364 to X. campestris NRRL B-1459 using the procedure described in Proc. Natl. Acad. Sci., USA Vol. 77 pp. 7347 to 7351 (1980) G. Ditta, et al. A mixture of the above mentioned bacterial strains and a third strain, E. coli JZ279 containing the plasmid pRK20l3 were incubated on a solid surface agar medium for 8 hours and subsequently deposited on the surface of M9 medium agar containing Kanamycin. This was incubated for 72 hours at 30°C and a resulting bacterial colony was isolated, designated NRRL B-1459 (pCHC3). Cultivation of this recombinant organism may be conducted under conditions identical to the non-recombinant organism, with the exception of the addition of Kanamycin to the solid surface medium for the purpose of distinguishing the plasmid containing organism from the plasmidless organism.

The fermentations were performed using the following medium:

| | |
|---|---|
| Corn Syrup | 35.0 g/l |
| $K_2HPO_4$ | 10.0 g/l |
| Soy Protein Concentrate | 2.1 g/l |
| $NH_4.NO_3$ | 0.2 g/l |
| $MgSO_4.7H_2O$ | 0.1 g/l |
| $FeSO_4.7H2O$ | 1 ppm |

One hundred milliliter aliquots of the fermentation medium were placed in 500 milliliter flasks, a seed inoculum of the test organism was aseptically transferred to the sterile medium, and cultivation was carried out at 30°C for 80 hours with orbital shaking.

The pH of the fermentation broth was adjusted to 4.5 and the broth was treated with the enzyme glucoamylase to hydrolyze residual corn syrup. The fermentation product was then recovered by precipitation with two volumes of isopropanol, dried, and the xanthan gum yield was determined gravimetrically (Table I). The initial and final concentration of corn syrup in the fermentation medium, measured as reducing sugar equivalents after hydrolysis with glucoamylase, were also determined.

## TABLE I

| Bacterial Strain | Yield (g/l) | Post-fermentation Reducing Sugar Conc. (g/l) |
|---|---|---|
| NRRL B-1459 | 25.3 | 3.2 |
| NRRL B-1459(pRK293)[a] | 25.8 | 3.2 |
| NRRL B-1459(pCHC3) | 28.5 | 2.6 |

[a]Insertion of the pRK293 plasmid in NRRL B-1459 was accomplished by substantially the same trans-conjugation procedure as for pCHC3.

As is seen, the strain containing pCHC3 produces a substantial increase in product yield over the strains containing the cloning vehicle alone or no pCHC3 plasmid.

EXAMPLE 3

**DEMONSTRATION OF THE INCREASED PYRUVATE CONTENT OF XANTHAN GUM PRODUCED BY X. CAMPESTRIS NRRL B-1459 CONTAINING THE PLASMID pCHC3**

To demonstrate the usefulness of this plasmid in increasing the pyruvate content of xanthan gum, the amount of pyruvate covalently attached to the polysaccharide was determined using the procedure described in "The Enzmatic Assay of Pyruvic and Lactic Acids: A Definitive Procedure", Clinica Chimica Acta Vol. 19, pp. 357 to 361 (1968) A. G. Hadjivassiliou and S. V. Rieder. The fermentation broth or purified xanthan solution (containing 0.2-0.4% w/v polysaccharide) was hydrolyzed with 0.1 N HCl for 4 hours. An 0.5 ml aliquot of the hydrolysate was mixed with 2.4 ml of 0.4 M triethanolamine (PH 8.0) and 0.1 ml of a NADH solution (2.5 mg/ml). The concentration of pyruvate is determined from the difference in the absorbance of the solution at 340 nm before and fifteen minutes after the addition of 0.02 ml of lactate dehydrogenase (200 units/ml) divided by the molar extinction coefficient of ADH ($\epsilon_{340} = 6.22 \times 10^3$). A comparison of the pyruvate content of the xanthan product produced by NRRL B-1459, containing no plasmid, containing the plasmid cloning vector, pRK293, or containing pCHC3, is shown in Table II.

## Table II

| Bacterial Strain | % Pyruvate (gm/100 gm product) |
|---|---|
| NRRL B-1459 | 3.8 |
| NRRL B-1459(pRK293) | 3.7 |
| NRRL B-1459(pCHC3) | 5.5 |

As is seen, the strain containing pCHC3 produces xanthan gum with a substantial increase in pruvate content over that produced by strains containing the cloning vector alone or no pCHC3 plasmid.

**Claims**

1. A double-stranded DNA fragment from the genome of a microorganism of the species Xanthomonas campestris, said fragment comprised of ca. 12.4 kb, having a restriction endonuclease map as depicted in Figure 1 and containing DNA sequences coding for genes which are essential for xanthan gum synthesis.

2. A recombinant DNA plasmid covalently containing therein the double-stranded DNA fragment as defined in Claim 1.

3. The recombinant DNA plasmid of Claim 2 covalently containing therein a) the double-stranded DNA fragment as defined in Claim 1 and b) a plasmid DNA vector selected from the group consisting of pRK290, pRK291, pRK292, pRK293, pRK310, pRK311, pKT210, pKT248, and pVK101.

4. A recombinant DNA plasmid of Claim 2 covalently containing therein a) the double-stranded DNA fragment as defined in Claim 1, and b) a plasmid DNA vector derived from the SalI cleavage of a gram-negative bacterial DNA plasmid, said plasmid DNA vector being capable of replication in Xanthomonas campestris.

5. The recombinant DNA plasmid of Claim 4 having the characteristics of the plasmid contained in the bacterial strain ATCC No. 53364.

6. The recombinant DNA plasmid pCHC3 having a restriction endonuclease map as depicted in Figure 2, obtainable from the microorganism E. coli (ATCC No. 53364).

7. A microorganism of the species Xanthomonas campestris, having the characteristics of ATCC No. 13951 (NRRL B-1459), having introduced therein at least one recombinant DNA plasmid as defined in Claim 2, and being capable of increased xanthan gum production by fermentation in the presence of an aqueous buffered nutrient medium containing assimilable sources of carbon, nitrogen and inorganic substances, as contrasted to the same microorganism in the absence of said recombinant DNA plasmid.

8. The microorganism of the species Xanthomonas campestris of Claim 7 wherein said recombinant DNA plasmid is pCHC3, obtainable from the microorganism E. coli (ATCC No. 53364).

9. The microorganism of the species Xanthomonas campestris of Claim 7 in biologically pure form.

10. The microorganism E. coli., containing plasmid pCHC3 and having ATCC No. 53364.

**Patentansprüche**

1. Doppelsträngiges DNA-Fragment von dem Genom eines Mikroorganismus der Art Xanthomonas campestris, bestehend aus circa 12,4 kb mit einer Restriktions-Endonuclease-Karte, wie in Figur 1 gezeigt, und enthaltend DNA-Sequenzen, die die Gene codieren, die für die Xanthangummisynthese wesentlich sind.

2. Rekombiniertes DNA-Plasmid, das kovalent darin (gebunden) das doppelsträngige DNA-Fragment wie in Anspruch 1 definiert enthält.

3. Rekombiniertes DNA-Plasmid nach Anspruch 2, das kovalent (gebunden) darin enthält: a) das doppelsträngige DNA-Fragment nach Anspruch 1 und b) einen Plasmid-DNA-Vektor ausgewählt aus der Gruppe bestehend aus pRK290, pRK291, pRk292, pRK293, pRK310, pRK311, pKT210, pKT248 und pVK101.

4. Rekombiniertes DNA-Plasmid nach Anspruch 2, das kovalent (gebunden) darin enthält: a) das doppelsträngige DNA-Fragment nach Anspruch 1 und b) einen Plasmid-DNA-Vektor, erhalten durch SalI-Spaltung des gram-negativen bakteriellen DNA-Plasmids, wobei der erwähnte Plasmid-DNA-Vektor zur Replikation in Xanthomonas campestris in der Lage ist.

**5.** Rekombiniertes DNA-Plasmid nach Anspruch 4 mit den Eigenschaften des in dem Bakterien - Stamm ATCC Nr. 53364 enthaltenen Plasmids.

**6.** Rekombiniertes DNA-Plasmid pCHC3 mit einer Restriktions-Endonuclease-Karte, wie in Figur 2 angegeben, erhältlich von dem Mikroorganismus E. coli (ATCC Nr. 53364).

**7.** Mikroorganismus der Art Xanthomonas campestris mit den Charakteristika von ATCC Nr. 13951 (NRRL B-1459), in den mindestens ein Rekombinations-DNA-Plasmid, wie in Anspruch 2 definiert, eingeführt ist und der zu einer erhöhten Xanthangummibildung durch Fermentation in Gegenwart eines wässrigen abgepufferten Nährmediums, enthaltend assimilierbare Quellen für Kohlenstoff, Stickstoff und anorganische Substanzen, in der Lage ist, im Gegensatz zu dem gleichen Mikroorganismus in Abwesenheit des Rekombinations-DNA-Fragments.

**8.** Mikroorganismus der Art Xanthomonas campestris nach Anspruch 7, wobei das Rekombinations-DNA-Plasmid pCHC3 ist, erhältlich von dem Mikroorganismus E. coli (ATCC Nr. 53364).

**9.** Mikroorganismus der Art Xanthomonas campestris nach Anspruch 7 in biologisch reiner Form.

**10.** Mikroorganismus E. coli enthaltend Plasmid pCHC3 mit der ATCC Nr. 53364.

**Revendications**

**1.** Fragment d'ADN à double brin provenant du génome d'un microorganisme de l'espèce Xanthomonas campestris, ledit fragment étant constitué d'environ 12,4 kb, possédant une carte d'endonucléase de restriction telle que représentée sur la figure 1 et contenant des séquences d'ADN codant pour les gènes qui sont essentiels à la synthèse de la gomme xanthane.

**2.** Plasmide à ADN recombinant contenant, lié par liaison covalente, le fragment d'ADN à double brin selon la revendication 1.

**3.** Plasmide à ADN recombinant selon la revendication 2, contenant, liés par liaison covalente, a) le fragment d'ADN à double brin selon la revendication 1, et b) un vecteur d'ADN de plasmide choisi parmi l'ensemble comprenant pRK290, pRK291, pRK292, pRK293, pRK310, pRK311, pKT210, pKT248 et pVK101.

**4.** Plasmide à ADN recombinant selon la revendication 2, contenant, liés par liaison covalente, a) un fragment d'ADN à double brin selon la revendication 1, et b) un vecteur d'ADN de plasmide dérivant du clivage par SalI d'un plasmide à ADN d'une bactérie gram-négative, ledit vecteur d'ADN de plasmide étant capable de subir une réplication dans Xanthomonas campestris.

**5.** Plasmide à ADN recombinant selon la revendication 4, ayant les caractéristiques du plasmide contenu dans la souche bactérienne ATCC No. 53364.

**6.** Plasmide pCHC3 à ADN recombinant ayant une carte d'endonucléase de restriction selon la figure 2, et que l'on peut obtenir à partir du micro-organisme E. coli (ATCC No. 53364).

**7.** Microorganisme de l'espèce Xanthomonas campestris, ayant les caractéristiques du microorganisme ATCC No.13951 (NRRL B-1459), dans lequel est introduit au moins un plasmide à ADN recombinant selon la revendication 2, et qui est capable d'augmenter la production de la gomme xanthane par fermentation en présence d'un milieu nutritif tamponné aqueux contenant des sources assimilables de carbone, d'azote et de substances minérales, par contraste avec ce même microorganisme en l'absence dudit plasmide à ADN recombinant.

**8.** Microorganisme de l'espèce Xanthomonas campestris selon la revendication 7, dans lequel ledit plasmide à ADN recombinant est le pCHC3, que l'on peut obtenir à partir du microorganisme E. coli (ATCC No. 53364).

**9.** Microorganisme de l'espèce Xanthomonas campestris selon la revendication 7, sous forme biologiquement pure.

10. Microorganisme E. coli, contenant le plasmide pCHC3 et ayant le No. ATCC 53364.

Figure 1

Figure 2